# EUROPEAN PATENT APPLICATION

(11) **EP 4 105 170 A1**
(43) Date of publication of application: **21.12.2022**
(21) Application number: 21290040.1
(22) Date of filing: 18.06.2021
(51) Int. Cl.: C01B 3/02, B01J 8/04, B01J 8/06, C01B 3/04, C01B 3/16, C01B 3/38, C07C 29/151, C10K 3/02

(54) **PROCESS AND PLANT FOR FLEXIBLE PRODUCTION OF SYNGAS FROM HYDROCARBONS**

(71) Applicant: Technip Energies France, 92741 Nanterre Cedex (FR)
(72) Inventor: Riegman, Jan-Jaap, 2700 AB Zoetermeer (NL); Walspurger, Stephane, 2700 AB Zoetermeer (NL); Jain, Prateek, 2700 AB Zoetermeer (NL)
(74) Representative: V.O.

(57) **Abstract**

Process for producing a chemical product, comprising
subjecting hydrocarbon feed and further reforming reactant to an endothermal reaction whereby a primary reformate is formed, in a primary fired heat-recuperating reformer reaction unit, comprising a catalyst zone and a primary reformate passage way arranged to transfer heat from said reformate to said catalyst zone;
optionally subjecting the primary reformate to a secondary reforming reaction, thereby forming a secondary reformate; and using primary reformate or second reformate as a heat exchange medium to supply reaction heat to an endothermal reaction, which endothermal reaction is carried out in a parallel heat-exchanger reactor.

## Description

The invention relates to a process for producing syngas from a hydrocarbon feed (1) and steam (2), comprising reacting the hydrocarbon and steam in a reformer reaction system . The invention further relates to a process for obtaining a component of syngas, e.g. hydrogen, from the produced syngas and to a process wherein the syngas or a component thereof is further processed to produce a further chemical product, such as ammonia, synthetic fuel or an alcohol, for instance methanol. The invention further relates to a chemical plant comprising a reformer reaction system.

Plants for producing syngas and other chemical products, such as ammonia, methanol or hydrogen, from hydrocarbons, in particular steam methane reforming (SMR) plants, are widely applied in refinery complexes, Amongst others, they supply hydrogen as a fuel or for upgrading of several products, for example in hydrocracking, hydrogenation or hydrodesulphurization. Additionally, hydrogen is used to produce ammonia, methanol and synthetic fuels, typically as a component of syngas (a mixture comprising hydrogen and carbon monoxide). As a by-product of the technology, CO₂ is produced and emitted to the atmosphere. The CO₂ is produced not only by combustion of a carbon-based fuel for heating the feedstock to the temperatures needed to carry out the reforming, but CO₂ is also formed as a side-product in the hydrogen production: the steam reforming reaction produces carbon monoxide (with methane as a starting compound: CH₄ + H₂O ⇄ CO +3 H₂), which is subsequently converted to carbon dioxide via the water gas shift reaction (CO + H₂O ⇄ CO₂ + H₂) in case additional hydrogen or less carbon monoxide in the product is required.

It is a general trend in the industry to research, develop and implement industrial processes having low carbon footprint. Traditionally syngas production for ammonia synthesis contributes to a large part of the industrial carbon dioxide emissions. Hydrocarbons such as natural gas, LPG, naphtha are applied as fuel as well as feedstock. Traditionally, the syngas production section comprises a fired primary reformer equipped with catalytic tubes and (optionally) a secondary reformer, where an oxidant is used. A secondary reformer is typically used to convert the remaining hydrocarbons, mostly methane, through partial combustion (4 CH₄ + O₂ + 2 H₂O → 10 H₂ + 4 CO). In the secondary reformer, such as an autothermal reformer (ATR), the feed is mixed with the oxidant in a burner zone, after which the partial oxidation of methane takes place in the combustion zone to produce carbon monoxide and hydrogen. Typically, a catalyst zone is present downstream of the combustion zone, where the gasses leaving the combustion zone attain thermodynamic equilibrium.

The outlet of the secondary reformer passes through heat exchangers to recover high grade heat from the process gas in the form of steam and to cool down the process gas upstream the water-gas shift section. For a typical ammonia production process, this process gas then reacts in the shift reactor(s) and substantial part of CO present in process gas gets converted to CO₂ and additional hydrogen, after which CO₂ is removed in CO₂ removal section resulting carbon dioxide concentration to ppm levels, followed by methanation that eliminates last traces of carbon oxides that are detrimental for the ammonia catalyst. The product gas, containing mostly nitrogen and hydrogen, is fed to the ammonia synthesis loop to product ammonia.

For a methanol plant, higher alcohols synthesis or a gas to liquids (GTL) plant, the optimal feed gas module is dependent on feedstock and choice of reforming technology. Advantageously two step reforming is employed, which includes primary reformer with an O₂ fired secondary reformer with an option of possible recycling of purge gas from the synthesis loop in case available.

In contrast with the ammonia secondary reformer that uses N₂ containing oxidant (such as air), secondary reformers used for providing syngas for the production of methanol and GTL, or hydrogen and other syngas derived products, traditionally use N₂ free oxidant, such as pure oxygen. This to limit the equipment size downstream the syngas generation unit and to avoid build-up of inert gasses in the downstream units. The process gas is compressed before entering the ammonia/methanol production loop. Traditionally CO₂ emission are largely coming from the firing in the primary reformer as well as external boiler to produce sufficient steam for a turbine driven compressor. With the initiatives to reduce the carbon-dioxide emissions, turbine driven compressors can be replaced with electrically driven compressors. This enables more heat integrated solution to drastically reduce the required carbon footprint as these solutions typically result in less steam production.

US 4,376,758 relates to an ammonia plant wherein a reformer system having a heat exchange reformer (2 streams in-1 stream out) in parallel to both a primary fired reformer and a secondary reformer (Figure 1 of US 4,376,758). Such arrangement is considered beneficial to obtain additional feedstock through an existing primary-secondary reformer units layout, or to reduce the size of the primary and secondary reformer units. Limitations of the technology include a limitation to the maximal capacity increase with the addition of heat exchange reformer. There is a need for an alternative process respectively plant configured for producing ammonia, in particular a process respectively plant that, allowing for decreasing the duty on the primary (fired) reformer or that offers another advantage, such as further described herein below.

In US2011/042620 a specific integration of a heat exchange reformer in an ammonia plant having a primary and a secondary reformer is described. The heat exchanger reformer is heated with the effluent of a secondary reformer and the reforming reaction is in parallel to the primary and secondary reformer. An objective of the specific integration is to increase the energy efficiency of the reformer while decreasing the heat flux, leading to a low severity and "low cost" primary reformer. The methane slip downstream the primary reformer is kept very high (>30mol%) compared to conventional primary reformer (mostly CH₄<20mol%). This is the consequence of using low outlet temperature (<640 °C), in the so-called "Lite reformer" which is a concept that minimizes the investment costs in primary reformer and minimize the use of natural gas when a low cost fuel used for firing the primary reformer. A major disadvantage of this approach is however the high methane slip remaining downstream the heat exchanger reformer.

US 5,181,937 shows the integration of a gas-heated reformer (GHR) type (2in-2out) heat exchange reformer with a primary and a secondary reformer where the GHR is placed upstream the primary reformer and receives the effluent of the secondary reformer (Figure 2 of US 5,181,937). This allows to significantly reduce the natural gas consumption in the primary reformer.

US 6,100,303 describes the integration of a GHR with a primary plus a secondary reformer configuration in a methanol plant. The process steam injected upstream the primary reformer is generated in the methanol loop. The stoichiometric Ratio (R) of syngas downstream the secondary reformer should be 2.2 to 2.5. The GHR is in parallel to the primary reformer, the effluent of the GHR is fed to the secondary reformer. The effluent of the secondary reformer is the heat source for the heat exchange reformer.

US2012/0149788 describes an arrangement in series of a GHR and SMR and then mixed feed (GHR-SMR effluent + fresh feedstock) to a partial oxidation reformer (POX), especially suited for very large methanol plants (>10000 MTPD).

All this prior art teaches on how to unload the duty of the primary-secondary reformers or the POX or ATR unit, however they do not allow for retrofit capacity expansion of the primary-secondary reformer.

WO2014/019610A1 describes a possibility to retrofit capacity expansion of the primary-secondary reformer in a reformer system: hydrogen production capacity in the primary-secondary reformer is increased by replacing the primary reformer tubes by a larger inner diameter while maintaining the same outer diameter of the tube by taking advantage of higher grade materials. The same philosophy is also known to be applied in the steam reformer for hydrogen and syngas plants. The increase in inner diameter of the tubes allows more flow through the tubes at the same pressure drop. The disadvantage of this technology is that the increase in capacity from the primary reformer also requires additional fired duty. Accordingly, apart from having to replace the catalyst tubes, typically additional modifications are required with respect to burners, heat recovery system and combustion air and flue gas fan.

US2007/051041 describes a method of an incremental increase of capacity expansion of up to 10% in an existing syngas based plant by providing an adiabatic pre-reformer and independent heater which is configured to receive a pre-reformer and preheated mixed feed stream from the pre-reformer and the heat of the pre-reformer and preheated mixed feed stream in order to reheat the temperature as high as 700°C. A downside of this method of capacity increase is that an additional difficult modification (high metallurgy, hydrogen service, high piping stresses) of the convection section heat recovery is required. Typically, the existing coils in the recovery section installed relatively close to each other, not allowing the installation of an additional coil. Furthermore, if the inlet temperature of the reformer is increased, this will also require changes of the reformer inlet system. Also a reactor with a dedicated pre-reforming catalyst is required.

US2014/171714 describes the option of increasing the hydrogen capacity of a steam methane reformer by applying a system of two pre-reformer reactors in series, where-in an oxygen stream is combined with the partially reformed outlet stream of the first-pre-reformer reactor, where the combined stream is introduced to the second pre-reformer reactor. This method results only in a minor capacity increase, with the advantage of not decreasing the steam production as a result of the additional heat recovery. A disadvantage of this system is that it requires many changes to the plant and adds additional partial oxidation steps.

WO2019220074A1 describes capacity increase in ammonia with the addition of a gas heated reformer (GHR) in parallel to a fired primary reformer or with a GHR replacing the fired primary reformer, with an adapted burner for the secondary reformer (with air/or only oxygen). A disadvantage of the process is that the capacity increase is dictated by the size of the secondary reformer (in the case of air as the oxidant supply).

As follows from the above described prior art, multiple ways to apply heat integration in the form of a parallel heat exchanger reformer as such are known to increase the capacity without significant modification to the primary and secondary reformer, however the capacity increase is limited by the design of the secondary reformer (that must ensure the required flow of oxidant is introduced to generate the synthesis gas at the desired ratio to compensate for the lower conversion in the heat exchanger reformer). Another major downside of known schemes making use of a heat exchanger reformer parallel to a primary fired reformer and secondary reformer is the lower outlet temperature of the parallel heat exchanger reformer compared to the secondary reformer, resulting in a relative higher methane slip.. This consequently has an impact on the downstream system since methane needs to be separated and recovered from the downstream applications. For example for ammonia production the methane slip to the synthesis loop is typically ∼0.3 vol% up to 0.6 (dry basis), but even down to 0.1 vol% (dry basis) is possible. The additional methane slip may build up (methane typically being an inert compound in the downstream synthesis loop) and this impacts sizing of the downstream equipment and compression power. Further, methane needs to be removed in a purge stream from the synthesis loop and generally is combusted. A higher methane slip thus typically also increases the CO₂ emissions. Secondly, the addition of a parallel heat exchanger reformer in ammonia synthesis adjusts the nitrogen to hydrogen ratio, which is typically around 3.0 mol/mol. In a revamp case the nitrogen to hydrogen ratio typically decreases, if no measures are taken. while with a newly build application the secondary reformer would have to be adjusted to maintain a N₂/H₂ ratio of ∼3.0mol/mol.

A continuing need exists to provide alternative processes and equipment to produce chemical products, in particular processes/equipment addressing one or more of the above indicated drawbacks, processes/equipment allowing a reduction in greenhouse gas emissions (carbon dioxide, methane). In particular, a continuing need exists to provide an efficient way to produce hydrogen, syngas or syngas derived products, such as ammonia, methanol, or synthetic fuels, from hydrocarbon feeds by reformation processes, whereby in particular the same production capacity can be maintained with a smaller reformer system or wherein production capacity can be increased without increasing the size of the reformer system. In particular, it would be desired not only to provide a way to realize one or more of these needs for new plants but also to provide convenient way to realize or more of these needs by refurbishment of existing plants.

It is an object of the present invention to address one or more of said needs. One or more alternative or additional objects which may be addressed follow from the description below.

The inventors now found that one or more objects of the invention are addressed by using a specific arrangement of the reformer system in combination with a specific type of reformer reaction units (such as reformer catalyst tubes).

Accordingly, the present invention relates to a process for producing a chemical product, comprising
subjecting a hydrocarbon feed and a further reforming reactant to an endothermal reforming reaction whereby a primary reformate is formed in a primary fired heat-recuperating reformer reaction unit (RT1), the primary fired heat-recuperating reformer reaction unit comprising a primary reformer catalyst zone (CZ1) and a primary reformate passage way (32, 34), downstream of the primary reformer catalyst zone and arranged to transfer heat from said reformate present in the primary reformate passage way to said primary reformer catalyst zone; optionally subjecting the primary reformate to a secondary reforming reaction, thereby forming a secondary reformate; and using primary reformate or second reformate as a heat exchange medium to supply reaction heat to an endothermal reaction, which endothermal reaction is carried out in a parallel heat-exchanger reactor (13).

In particular, the present invention relates to a process for producing a chemical product, comprising
subjecting a hydrocarbon feed (1) and a further reforming reactant (2,3) - typically selected from the group consisting of steam, carbon dioxide and mixtures thereof - to an endothermal reforming reaction whereby a primary reformate is formed, which endothermal reforming reaction is carried out in a primary fired heat-recuperating reformer reaction unit (RT1), comprising a reformer catalyst zone (CZ1) and a primary reformate passage way (32, 34) downstream thereof, which passage way is arranged to transfer heat inside the primary reformer reaction unit (RT1) from the primary reformate whilst present in the primary reformate passage way (32, 34) to the primary reformer catalyst zone (CZ1) before the primary reformate leaves the primary reformer reaction unit via a primary reformate outlet (33);
optionally subjecting the primary reformate to a secondary reforming reaction, thereby forming a secondary reformate; and using primary reformate or second reformate (10) as a heat exchange medium to supply reaction heat to an endothermal reaction, which endothermal reaction is carried out in a parallel heat-exchanger reactor (13) comprising an endothermal reaction zone (CZP) wherein one or more reactants (11, 12) are subjected to an endothermal reaction, and which parallel reactor further comprises a heat-exchanging medium passage way (HEP) through which the primary or secondary reformate passes and which passage way is arranged to transfer heat from the reformate present in the heat-exchanging medium passage way (HEP), to the parallel endothermal reaction zone (CZP) inside the parallel endothermal reactor (13).

In particular, the invention provides a process for producing a chemical product from a hydrocarbon feed (1) and a further reforming reactant (2,3) selected from the group consisting of steam, carbon dioxide and mixtures thereof, , comprising reacting the hydrocarbon and the further reforming reactant in a reformer reaction system, wherein the reformer reaction system comprises a primary reformer (6), into which a first part of the hydrocarbon feed and a first part of the further reforming reactant are fed (4), and a parallel reformer (13), into which a second part of the hydrocarbon feed and a second part of the reformer reactant are fed (11),
wherein the primary reformer (6) is a fired heat-recuperating reformer, comprising a radiant section (RS) provided with burners (B), wherein fuel (5) is combusted to provide heat to the said first part (4) of hydrocarbon and further reformer reactant, and a primary heat-recuperating reformer reaction unit (RT1), located in the radiant section (RS),
which primary reformer reaction unit (RT1) comprises a primary reformer catalyst zone (CZ1), containing a reformer catalyst, into which catalyst zone said first part (4) of the hydrocarbon feed and further reformer reactant are fed and in which catalyst zone the fed hydrocarbon and further reformer reactant - react in the presence of the catalyst, whereby a primary reformate is formed,
which primary reformer reaction unit (RT1) further comprises
a primary reformate passage way (32, 34), downstream of the primary reformer catalyst zone (CZ1), through which passage way (32, 34) the primary reformate passes and which passage way (32, 34) is arranged to transfer heat inside the primary reformer reaction unit (RT1) from the primary reformate whilst present in the primary reformate passage way (32, 34) to the primary reformer catalyst zone (CZ1) before the primary reformate leaves the primary reformer reaction unit via a primary reformate outlet (33); and wherein
the parallel reformer is a heat-exchanger reformer (13), comprising a parallel reformer catalyst zone (CZP) into which the second part of the hydrocarbon feed and the second part of the steam (11), plus optionally additional further reformer reactant (12), are fed, and in which parallel reformer catalyst zone (CZP) the fed hydrocarbon and further reformer reactant - react in the presence of a reformer catalyst present in the catalyst zone (CZP) whereby a parallel reformate is formed, the parallel reformer (13) further comprising a heat-exchanging medium passage way (HEP), arranged to transfer heat from a heat exchange medium present in the heat-exchanging medium passage way (HEP), to the parallel reformer catalyst zone (CZP) inside parallel heat-exchanger reactor (13), wherein the heat exchange medium is primary reformate (7) from the primary reformer or a secondary reformate (10), obtained after subjecting primary reformate to further processing, in particular a secondary reforming reaction.

In a process according to the invention, the reactant or reactants fed into the parallel heat-exchanger reactor (13) can be the same or different from the reactants fed into the primary fired reformer (6). If one or more reactants are used that are the same, the composition of the reaction mixture can be the same or different. E.g. in case hydrocarbon feed and further reactants for a reforming reaction (such as steam, carbon dioxide) are also fed into the parallel reactor, the first part of the of the hydrocarbon feed, i.e. the part fed to the primary reformer (6) can have the same or a different composition as the second part of the hydrocarbon feed, i.e. the part fed to the parallel heat-exchanger reactor (6). Likewise, the first part of the further reforming reactant, i.e. the part fed to the primary reformer (6), and the second part of the reformer reactant, i.e. the part fed to the parallel heat-exchanger reactor can be the same or different.

Further, the invention relates to a chemical plant, suitable for carrying out a process according to the invention. Usually, the chemical plant according to the invention, comprises a reformer reaction system for producing a product from a hydrocarbon feed (1) and a further reformer reactant, (2), such as steam or carbon dioxide, the reformer reaction system, comprising a primary reformer (6), the plant further comprising a parallel heat-exchanger reactor configured to carry out an endothermal reaction (13), preferably a heat-exchanger reformer (13),
wherein the primary reformer (6) is a fired heat-recuperating reformer, comprising a radiant section (RS), wherein burners (B) and a primary reformer reaction unit (RT1) are present, which primary reformer reaction unit (RT1) comprises a primary reformer catalyst zone (CZ1), containing a reformer catalyst and an inlet for feeding hydrocarbon feed and further reformer reactant into the primary reformer catalyst zone (CZ1), which primary reformer reaction unit (RT1) further comprises a primary reformate passage way (32, 34), downstream of the primary reformer catalyst zone (CZ1), configured to receive primary reformate formed in the primary catalyst zone and allow passage of said primary reformate through the passage way (32, 34) and which passage way (32, 34) is arranged to internally transfer heat from the primary reformate in said passage way (32, 34) to the primary reformer catalyst zone (CZ1) and a primary reformate outlet (33) downstream of the passage way (32, 34) arranged to internally transfer heat to the primary reformer catalyst zone (CZ1); and wherein
the parallel heat-exchanger reactor (13), comprising a parallel reaction zone, preferably a catalyst zone (CZP), comprising an inlet for reactants, e.g., hydrocarbon feed and steam, carbon dioxide or a mixture thereof (11), plus optionally an additional reactant inlet (12),
the parallel heat exchanger reactor (13) further comprising a heat-exchanging medium passage way (HEP), arranged to transfer heat from a heat exchange medium present in the heat-exchanging medium passage way (HEP), to the parallel reaction zone (CZP) inside the parallel heat exchanger reactor (13) wherein the heat exchange medium passage way has an inlet for heat exchange medium in fluid communication with the outlet (33) of the primary reformer reaction unit via a passage way (7, 9, 10), which optionally contains one or more processing units, e.g. a further reformer, configured to treat primary reformate, e.g. process primary reformer into a secondary reformate (10), obtained after subjecting primary reformate to further processing, in particular a secondary reforming reaction.

Advantageously, a chemical plant according to the invention is configured for carrying out a process according to the invention.

The invention in particular relates to a process according to any of the claims 1-17 respectively a plant according to any of the claims 18-21.

Good results are in particular achieved with a parallel heat exchanger reformer (13). Alternatively or in addition another type of endothermal reaction or reactor can suitably be employed, in particular reverse water gas shift reactions to produce carbon monoxide or syngas from hydrogen and carbon dioxide; ammonia decomposition, for example for production of hydrogen; methanol decomposition; for example for production of hydrogen and/or syngas; and fusel oil reforming to produce hydrogen; respectively reactors configured for any of these reactions.

Good results are achieved in particular with steam as a further reformer reactant in the primary reformer, the parallel reformer (if used) or both. Alternatively, instead of the addition of steam (3), part of the steam or the complete steam quantity for the primary reformer, the parallel reformer (if used) or both can be replaced with carbon dioxide..

The invention is described more fully herein with reference to the accompanying drawings, in which embodiments of the invention are shown, including some optional elements, e.g. a secondary reformer unit (9) or additional supply (12) for further reformer reactant, such as a steam supply. Also locations of units and process lines may deviate from what is schematically shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. Relative terms as well as derivatives thereof should be construed to refer to the orientation as then described or as shown in the drawing under discussion. These relative terms are for convenience of description and do not require that the system be constructed or operated in a particular orientation unless stated otherwise.

The skilled person will be able to design and operate suitable operational units of the hydrogen plant or used in a process according to the invention, using the present disclosure in combination with common general knowledge and optionally one or more of the documents cited herein. E.g. the skilled person will be able to provide suitable process/plant units (e.g. a reformer units, shift reactor zone units, carbon dioxide recovery units, hydrogen recovery units, heat exchanger units, systems configured to produce ammonia, methanol, synthetic fuel or another chemical product) and passage ways, e.g. pipes, lines, tubes, conduits or other channels for passing fluids (such as gases, liquids, mixtures thereof) from one processing unit to another, directly or indirectly, based on the present disclosure, the cited documents and common general knowledge.

For the purpose of clarity and a concise description, features are described herein as part of the same or separate embodiments, however, it will be appreciated that the scope of the invention may include embodiments having combinations of all or some of the features described.

As used herein, the singular forms "a", "an" and "the" are intended to include the plural forms as well -, e.g. "a reformer reaction unit" includes "reforming reactor units"; " a burner" includes "a plurality of burners", etc, unless the context clearly indicates otherwise. The term " or" includes any and all combinations of one or more of the associated listed items, unless the context clearly indicates otherwise (e.g. if an "either ....or" construction is used). It will be understood that the terms "comprises" and "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise, it will be understood that when a connection between structures or components is described, e.g. a passage way, this connection may be established directly or through intermediate structures or components unless specified otherwise.

The term "(at least) substantial(ly)" or " (at least) essentiall(ly)" is generally used herein to indicate that it has the general character or function of that which is specified. When referring to a quantifiable feature, this term is in particular used to indicate that it is at least 75 %, more in particular 90 % or more, even more in particular 95 % or more of the maximum of that feature. The term 'essentially free' is generally used herein to indicate that a substance is not present (below the detection limit achievable with analytical technology as available on the effective filing date) or present in such a low amount that it does not significantly affect the property of the product that is essentially free of said substance. In practice, in quantitative terms, a product is usually considered essentially free of a substance, if the content of the substance is 0 - 1 wt.%, in particular 0 - 0.5 wt.%, more in particular 0 - 0.1 wt.%; as will be understood by the skilled person, in particular for catalyst-contaminants, a content of the contaminant may need to be significantly lower than 0.1 wt.% for a product to be essentially free thereof, in particular less than 10 ppm by weight (ppmw), less than 1 ppmw or less than 0.1 ppmw.

In the context of this application, the term "about" or "close to" includes in particular a deviation of 10 % or less from the given value, more in particular 5% or less, more in particular 3% or less.

The term "process gas" is in particular used for a gas obtained in the process or plant according to the invention from the hydrocarbon feed and water (steam). Typically process gas is a valuable gas, as an intermediate product or end-product, unlike flue gas. Typically, the term process gas is used herein for a syngas, obtained in a reformer or a syngas obtained from a reformate having been further processed, e.g. in a shift reactor.

A reformate generally comprises H₂, CO, CO₂, usually water and usually methane. Water is generally present if steam has been used as a further reactant for the reforming reaction, but may also form in a dry reforming process. Further inert gas (i.e. inert in the reformers) may be present in particular nitrogen.

Shift reactor process gas is the gas obtained after subjecting the reformate (which may have been further processed before being fed to the shift reactor zone) to a reaction in the shift reactor. It typically comprises H₂, CO, CO₂, water and usually methane. Further in particular nitrogen may be present.

The term 'higher alcohol' refers to any alcohol having at least 2 carbon atoms. In particular, the term is used for mono-alcohols, di-alcohols and polyols having 2 - 20 carbon atoms, preferably 2 - 14 carbon atoms, more preferably 2-10 carbon atoms, in particular 2-6 carbon atoms. In a particularly preferred embodiment, the higher alcohol is ethanol, a propanol, a butanol, a methylpropanol, a pentanol or a hexanol.

### BRIEF DESCRIPTION OF THE FIGURES:

Figure 1 schematically shows a conventional reformer system for a reformer process/plant (Case 1, below)
Figure 2 schematically shows a conventional reformer system for a reformer process/plant (Case 2, below)
Figure 3 schematically shows a reformer system for a process/plant according to the invention (e.g. Case 4, below)
Figure 4 schematically shows two preferred configurations of heat-recuperating reformer reactor units; only a single reformer tube is shown, in practice general a plurality of those (in parallel) is provided (in case of a fired reformer reaction unit: a plurality of tubes in a single radiant section).

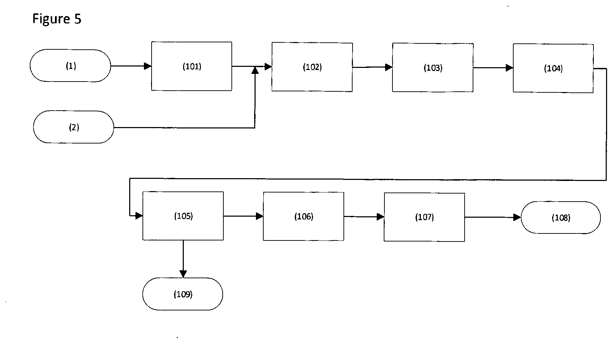

Figure 5 schematically shows a block scheme of an exemplary process according to the invention, including a pre-treatment zone for pre-treatment of the hydrocarbon feed (e.g. desulphurization, olefin saturation), the reforming section (wherein primary reforming, the optional secondary reforming and the parallel reforming take place), a shift reactor section, downstream processing of the process gas (syngas) and the chemical synthesis section, wherein a further product can be produced from the syngas or a component thereof (such as ammonia, an alcohol, etc).
Figure 6 schematically shows to examples of heat-exchanger reformers, suitable for use as a parallel reformer in accordance with the invention.

The present invention allows reducing the carbon-dioxide emissions from the syngas production with a combination of a primary and preferably a secondary reformer (such as an autothermal reformer) significantly by applying both parallel reforming heat exchanger reactor as well as recuperative reforming inside the primary reformer. The process and plant are described below in more detail and is applicable for ammonia production, methanol production, higher alcohol production, dimethyl ether production, synthetic fuel production, hydrogen production, hydrogen rich gas production (for example mixture of hydrogen and nitrogen) or syngas production, but is not limited to these applications.

The process and plant design allows easy adaptation of process operating conditions to adjust the composition of the (combined) reformate after the final reforming step dependent on the preferred composition for the synthesis of a chemical product of interest, which facilitates alternating between different chemical production processes downstream of the reformer system. The invention is both suited to be applied by refurbishing an existing plant and for newly build plants.

The reduction in CO₂ emissions of the syngas generation units is accomplished in particular by effectively unloading the firing demand of the primary reformer due to the application of recuperative reforming to increase the throughput in the primary reformer while not increasing or increasing very modestly the duty of the fired equipment. In an advantageous embodiment, the use of recuperative reforming reactor units, in particular recuperative reforming reactor tubes allows for a reduction size of the reformer of about 20% in an existing primary reformer, compared to conventional reformer tubes, while simultaneously reducing the firing demand by about 20%. Dependent on the circumstances a smaller or higher reduction is feasible.

Thus, the present invention allows to use the advantage of such recuperative reformer reaction units by either maintaining a catalyst outlet temperature that is equivalent to the catalyst outlet temperature of a conventional tube or increasing the catalyst outlet temperature while the internal heat recovery ensure a lower primary reformer outlet temperature and consequently a lower temperature of the process gas between the primary reformer and the secondary reformer.

Placing a process-gas-heated reformer in parallel with the primary reformer will allow for a further reduction in firing to produce the same syngas production rate. Additionally, the unloading of the duty of the primary reformer by recuperative reforming allows more oxygen addition in the secondary reformer (when present) resulting in a lower methane slip, while the parallel gas-heated reformer produces additional hydrogen. The combination of reformer technologies in accordance with the present invention overcomes limitations experienced in the separate technology implications.

Moreover, the combination of recuperative reforming with a parallel process-gas-heated reformer unexpectedly results into a significant reduction of the required fired duty in the primary reformer of -typically - up to about 50 %, although a higher reduction is feasible at least in some embodiments. Thus, combination of recuperative reforming in a primary heated reformer with heat exchanger reformer parallel to the primary reformer, or - when a secondary reformer is employed - parallel to both primary and secondary reformer can achieve simultaneously a significant reduction in direct CO₂ emissions (from fired heaters) and oxidant requirement (for the secondary reformer) as well as providing an advantageous syngas composition for multiple downstream production of chemical products, including but not limited to hydrogen, synthetic fuels, ammonia and methanol.

In an advantageous embodiment, the process/plant is a hybrid process/plant wherein alternatingly or simultaneously process gas obtained in the reformer system is fed into different reactors or other processing units for producing a chemical product of interest, in particular two or more products selected from the group consisting of ammonia, methanol and synthetic fuels.

Next, processes and plants according to the invention are described in further detail.

The hydrocarbon feedstock (1) fed into the reformer system (102) can be any hydrocarbon feedstock suitable for being subjected to reformation by reaction with water, carbon dioxide or a mixture thereof. It can in particular be a feedstock wherein the hydrocarbon is a feedstock at least substantially consisting of methane, such as natural gas or a biogas-based methane stream, or a fuel gas obtained from an ethylene plant (typically obtained from de-methanizer); propane gas (LPG), naphtha or (bio)refinery off-gas.

Dependent on the purity of the feedstock, the feedstock may be subjected to a pre-treatment in a pre-treatment section (101) prior to being fed into the primary reformer, such as to a hydrodesulphurization. Pre-treatments, conditions therefore and suitable pre-treatment units, may be based on known technology. In particular, when using a pre-treatment such as hydrodesulphurization, a make-up stream comprising hydrogen is usually added to the feed to ensure purification of the feed in the hydrodesulphurization section. A stream comprising hydrogen produced in a process according to the invention can be used to that purpose.

The hydrocarbon feedstock (1) is mixed with the further reformer reactant selected from steam, carbon dioxide and mixtures thereof (3), providing a mixture (4) that is fed into reforming section (102), in particular into the recuperative reformer reaction unit (RT1) of the fired reformer (6)). Optionally, the reformer system comprises a pre-reformer, upstream of the fired reformer (6), and - if present -upstream of the parallel heat exchanger reformer outside the radiant section (RS). The use of one or more pre-reformer units, usually one or more adiabatic pre-reformer units, to partially perform the reforming reaction (before preheating the pre-reformed mixture to the inlet temperature of the main reformer), is advantageous to unload the duty of the reforming reaction. The use of steam as a further reformer reactant is generally known in the art, and described also in detail in the above cited prior art. Alternatively, instead of the addition of steam (3), part of the steam or the complete steam quantity can be replaced with carbon dioxide. Resulting in a reforming section feed (4) consisting of the hydrocarbon feedstock, carbon dioxide and possibly steam. The use of carbon dioxide for the reforming reaction is known in the art ad dry reforming (DRM). The skilled person will be able to determine suitable conditions based on the teaching in the present disclosure, common general knowledge and e.g. Mohamad H A, A mini-review on CO₂ Reforming of Methane, Progres Petrochem Sci. 2(2) PPS.000532.2018.

The mixture to be fed into the reformer system usually at least substantially consists of hydrocarbon and the further reformer reactant (steam, carbon dioxide or a mixture thereof). The further reformer reactant and hydrocarbon feed may be fed in ratio's known in the art. In particular if steam is used as the major the sole further reformer reactant, usually the ratio hydrocarbon to steam fed into the reformer reaction unit is at least 2.0 mol/mol, preferably at least 2.5 mol/mol, in particular about 3.0 mol/mol or more. Usually the ratio hydrocarbon to steam fed into the reformer reaction unit is 5.0 mol/mol or less, preferably 4.0 mol/mol or less, in particular about 3.0 mol/mol or less. A ratio of hydrocarbon to steam ratio of 2.5 to 3.0 mol/mol is generally preferred as this results in the minimized hydrocarbon consumption and CO₂ emissions. In particular if carbon dioxide is used as essentially the sole further reformer reactant, the ratio of hydrocarbon to CO₂ usually is at least about 2 mol/mol, preferably at least about 2.5 mol/mol, in particular about 3.0 mol/mol or more. In particular if carbon dioxide is used as essentially the sole further reformer reactant, the ratio of hydrocarbon to CO₂ usually is 6 mol/mol or less, preferably 5 mol/mol or less, in particular about 3 mol/mol. If a mixture of further reactions is used, suitable and preferred ratios can be calculated based on the ratio steam/CO₂ and the above ratios, wherein about stoichiometric ratios are particularly preferred.

The temperature of the hydrocarbon and further reformer reactant, (a gaseous mixture), preferably steam or a gas at least substantially consisting of steam, at the inlet (30) of the (catalyst zone CZ1 of) the heat recuperating primary reformer reaction unit (such as a heat recuperating reformer reaction tube) generally is in the range of about 350 to about 700 °C. Typical preferred temperatures depend on the hydrocarbon feedstock and the overall optimization of the plant. In general, without a prereformer the inlet temperature is between 500 and 600°C and with a pre-reformer a temperature at the inlet of the (catalyst zone CZ1 of) the heat recuperating primary reformer reaction unit is typically at least 500 °C, preferably up to 650 °C .

As the reaction in the primary reformer is highly endothermic, the heat required for the reaction is typically supplied by firing of a fuel (5). The preferred fuel is hydrogen or hydrogen-rich gas. In an embodiment, such hydrogen or hydrogen-rich gas or part thereof has been produced in the process and recovered from the reformate or a process gas obtained from the reformate. This recovery can be accomplished in a known way, e.g. by temperature swing adsorption, pressure swing adsorption or a hydrogen selective membrane separator. It is also possible to use a hydrocarbon fuel to provide the heat or a part thereof.

Traditionally the catalyst in the catalyst zone is pellet based, while recent development also applies structured catalysts. The outlet temperature of the primary catalyst zone (CZ1) is generally in the range of about 600 to about 1000°C. For a process wherein use is made of a secondary reformer unit, wherein primary reformate is reacted with an oxidant, the temperature at the outlet of the primary catalyst zone (CZ1) is advantageously between 700 and 850°C. For a process without the secondary reformer, the temperature at the outlet of the primary catalytic zone (CZ1) is advantageously above 900°C.

The gas from the outlet of the primary catalytic zone (CZ1) is applied to provide part of the heat of reaction allowing the temperature of the primary reformate exiting the primary reformer reactor unit (33),to be significantly lower than the outlet temperature of the catalytic zone CZ1. Typically the primary reformate from the primary reformer reactor unit is at least 50°C and preferably more than 100°C lower than the outlet temperature of the catalytic zone (CZ1) and at least 30-100°C higher than the inlet temperature of the primary reformer unit.

The internal heat recovery significantly decreases the required fired duty of the radiant section of the primary reformer (6) and subsequently reduces the need for externally applied fuel (5) and eventually the associated CO₂ emissions.

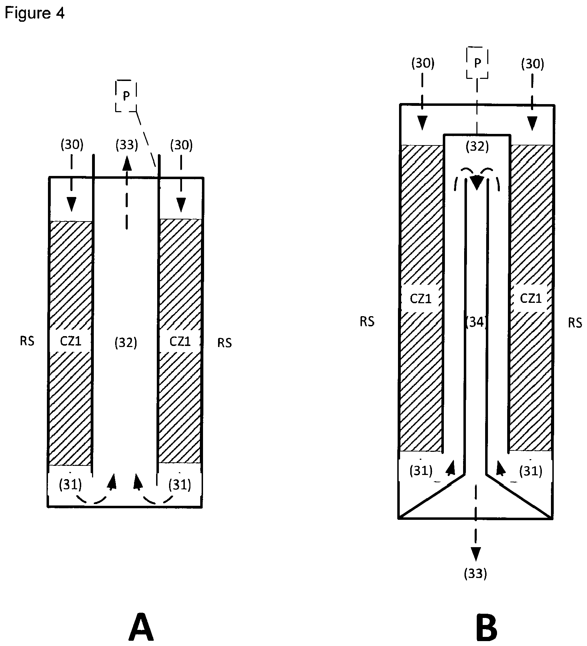

The present invention makes use of a heat-recuperating reformer reaction unit, such as a heat-recuperating reformer catalyst tube. A particularly suitable design of such unit is known from WO-2018/077969. In recuperative reforming, heat is recovered internally from the primary reformate inside the reformer reaction unit. Advantageously, the heat-recuperating reformer reaction unit (RT1), comprises an outer reactor channel (comprising catalyst zone CZ1) and an inner channel (primary reformate passage way, 32, 34) configured to exchange heat with the outer reactor channel, said heat recovery extending coaxially inside the outer reactor channel; said inner and outer channel together are also referred to in the art as forming a reformer reaction unit (RT1), typically a reformer tube. At least a substantial part of the inner channel (32) and at least a substantial part of the outer channel are separated via a heat-conductive partition (wall), allowing heat transfer from the inner channel (reformate passage way 32, 34) to the outer channel (catalyst zone CZ1) by heat conduction for at least a substantial part, via said partition (P). The outer reactor channel (providing a passage way between inlet 30 and inner channel 32 for gas) generally contains a catalyst, usually a catalyst bed (CZ1) catalysing the reaction between the hydrocarbon and the water (steam) under formation of the primary reformate. The outer reactor channel has a feed inlet (30) via which, during use, the hydrocarbon and the water (steam) are brought in contact with the catalyst (fed through the bed) (CZ1) and an outlet (31) for primary reformate, which inlet and outlet are located at opposite ends of the outer channel containing the catalyst. During use, the reformate is fed from outlet (31) into the inner channel (32, 34). Heat is then transferred from the reformate flowing through the inner channel to the contents of the outer reactor channel and reformate leaves the heat-recuperating reformer unit via a gas outlet (33). Figure 4 schematically shows two concepts of preferred recuperative reforming reaction units. Heat is supplied partially by the internal heat recovery, wherein heat is directly transferred from primary reformate inside the internal channel (32, 34) of the reforming reaction unit through the heat-conductive partition (wall) between the internal channel and the outer channel comprising the catalyst into the contents of the outer channel (hydrocarbon/steam mixture being subjected to the reforming reaction). Thus this internal heat transferred is without transfer to another gaseous medium outside the reformer reaction unit such as flue gas in the radiant section or in a convection section. A further part of the required heat for the endothermic reforming reaction is provided by firing of fuel in the radiant section (RS in Figure 4).

Concept A in Figure 4 (left hand) shows a reformer tube with a single pass, where the inlet and outlet of the gas is at the same side of the tube. The hot reformed gas flows counter-currently (32) back and cools down the gas by supplying heat to the reacting gas and reformer product in the catalyst zone (CZ1) in the outer channel.

Concept B of Figure 4 (right hand) adds a third pass where the cooled gas flows counter currently in an additional innermost channel (34), typically cocurrent with flow in the primary reformer catalyst zone (outer channel) to exit the reformer tube at the opposite side of the inlet (33).

Suitable reformer catalysts are generally known in the art. Particularly suitable is a nickel catalyst (a catalyst comprising metallic nickel or nickel oxide), which usually is provided on a ceramic support, e.g. alumina. The catalyst in a heat-recuperative reformer reaction unit preferably is a structured catalyst. Examples of structured catalysts suitable for use in a steam reforming process or hydrogen plant in accordance with the invention are known per se. The catalyst preferably has an annular configuration. An advantage of an annular configuration is the ability of the reforming tubular reactor to process higher feedstock flow rate due to large effective surface area and low pressure drop design and therefore allows for avoiding the increase in size of the steam reformer, compared to the conventional reforming process.

Advantageously, the catalyst structure is pre-formed into an annular structure or composed of several pre-formed parts, together forming an annular structure. Advantageous catalyst structures for a reformer reaction system in accordance with the invention can be based on the contents of PCT/EP2020/068035 of which the contents are incorporated by reference, in particular the claims and figures. Thus, in an advantageous, the heat recuperative reformer comprises a catalyst tube assembly, comprising
- an outer reactor tube having an inlet end and an outlet end opposite the inlet end, and including an inwardly protruding element;
- a centering assembly including an inner tube having an inlet end and an outlet end;
- a tubular boundary having a closed end and an open end;
wherein the tubular boundary is configured to extend substantially coaxially within the outer reactor tube and substantially coaxially around the inner tube, such that the catalyst tube assembly includes a first annular channel between the outer reactor tube and the tubular boundary, and a second annular channel between the tubular boundary and the inner tube, wherein the second annular channel is in fluid connection with the first annular channel near the open end of the tubular boundary, and in fluid connection with the inner tube at the closed end of the tubular boundary, wherein the outlet end of the inner tube includes at least one sealing member configured to be in sealing engagement with the inwardly protruding element of the outer reactor tube.

The structured catalyst may be a catalytic material coated on a monolith or corrugated plate, enhancing the heat transfer properties in the inside of the tube. Examples are shown in e.g. , US2010/0254864. Examples of annular catalysts in a recuperative reformer tube are shown in WO 97/26985. The annular reactor consists of a U-tube reactor (or Bayonet type reactor) that contains a riser tube in its central part. The catalyst is arranged in the annular space and the process gas flows back upwards in the central riser. The process gas is collected on the top side . Both process gas inlet and outlet system are therefore on the top side of the reactor assembly. WO 97/26985 also shows a tube surrounding the U-tube reactor (or Bayonet type reactor) where the combustion flue gas are circulated and provide the heat necessary for the reforming reaction. The combustion occurs in an externally located burner and the flue gas are brought in contact with the reactor via a jacketed cylindrical chamber surrounding the reactor.

A further, example is shown in US2007/0025893. It shows a reactor design with stackable structures placed in annular configuration inside the reforming reactor.

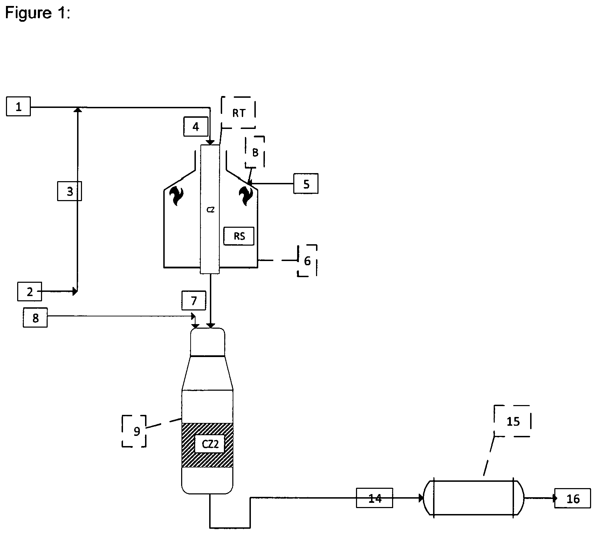

Further, the recuperative reformer reaction unit may e.g. be based on EP-A 0 725 675, US 5,162,104, US 5,219,535 or WO 2018/077969. The recuperative reformer reaction unit is advantageously based on a catalyst tube design described in WO2018/077969 of which the contents, in particular the claims and the figures, are incorporated by reference. Thus, with reference to claim 1, Figure 1 of WO2018/077969 and present Figure 4B , advantageously the recuperative reformer reaction unit comprises a catalyst tube for regenerative catalytic conversion of process gas in an industrial furnace comprising
- a catalyst tube inlet for gaseous hydrocarbon feed to enter the catalyst tube and a catalyst tube outlet for reformate to exit the catalyst tube, which inlet and outlet are located at opposite ends of the catalyst tube;
- an outer reactor tube;
- an inner tube that extends coaxially inside the outer reactor tube;
- a boundary located between the inner wall of the outer reactor tube and the outer wall of the inner tube;
- a first annular channel for catalytically converting the hydrocarbon feed in the presence of water, which channel is defined by the inner wall of the outer reactor tube and the outer wall of the boundary, which channel contains the catalyst;
- a second annular channel for reformate to flow counter-currently or co-currently to the feed flowing through the first annular channel, which second annular channel is defined by the inner wall of the boundary and the outer wall of the inner tube ;
- an inlet barrier at the inlet end of the catalyst tube for preventing gas to exit the outer reactor tube from the second annular channel and inner tube (at the inlet end of the catalyst tube;
- an outlet barrier at the outlet end of the catalyst tube for preventing gas to exit the outer reactor tube (1,11) from the first annular channel and from one of the second annular channel and the inner tube, while allowing gas to exit the outer reactor tube from the other of the second annular channel and the inner tube;
wherein the inner tube, first annular channel and second annular channel each have an opening at the inlet side of the catalyst tube and an opening at the outlet side of the catalyst tube,
wherein the catalyst tube inlet is fluidly connected with the opening of the first annular channel at the inlet end of the catalyst tube; the opening of the first annular channel at the outlet end of the catalyst tube is fluidly connected with either the opening of the second annular channel at the outlet end of the catalyst tube or the opening of the inner tube at the outlet end of the catalyst tube; the opening of the second annular channel at the inlet end of the catalyst tube is fluidly connected with the opening of the inner tube at the inlet end of the catalyst tube; and either the opening of the inner tube at the outlet end of the catalyst tube or the opening of the second annular channel at the outlet end of the catalyst tube is fluidly connected with the catalyst tube outlet.

In accordance with the invention, usually a secondary reformer is provided, downstream of the primary reformer although this secondary reformer can be omitted if desired in various applications; e.g. where the outlet temperature of the primary reformer is higher than 900°C.

The secondary reformer is typically a reformer wherein an oxidant, typically oxygen, is reacted with primary reformate. The oxidant is typically provided as a gas, usually comprising at least 10 vol % oxygen; such as pure oxygen gas (for instance at least 99 vol% purity), air, oxygen-enriched air (typically up to 50 vol % oxygen), or even air having a reduced oxygen content (oxygen-depleted air, typically at least 10 vol % ogygen).

Suitable secondary reformers such as autothermal reformers (ATR) or partially oxidation reformers (POX) are generally known in the art and also described in the prior art described above.

Typically, in the secondary reformer, such as an ATR, hydrocarbon the first reformate or part thereof is mixed with the oxidant supplied through a channel in the internal mixing zone, after which the partial oxidation of methane takes place in the combustion zone to produce carbon oxides and hydrogen. Usually for an ATR, downstream of the combustion zone, the process gas passes a catalyst zone where the process gas typically attains a composition close to thermodynamic equilibrium. At the outlet of the secondary reformer, in particular the ATR, the product reformer advantageously essentially consists of hydrogen, carbon monoxide and carbon dioxide, excess steam from the reaction (if steam has been used as a further reformer reactant) and optionally nitrogen (if nitrogen is present in the gas comprising oxidant or the feed) and a minor amount of unconverted methane (typically up to 0.3 vol%, in particular 0.1-0.3 vol %).

The secondary reformer (9) is arranged to subject the primary reformate or part thereof to a secondary reforming reaction, wherein the primary reformate reacts with an oxidant, typically supplied as a gas comprising the oxidant, preferably a gas comprising oxygen, whereby a secondary reformate is formed and feeding the secondary reformate (10) or part thereof into the heat-exchanging medium passage way (HEP) where it transfers heat to the parallel reformer catalyst zone (CZP). The oxidant is fed into the secondary reformer reaction zone, usually as a gas comprising the oxidant (e.g. air or pure oxygen), via a channel 8. The preferred gas comprising oxidant is depending on the downstream application of the syngas produced. For ammonia synthesis the preferred gas comprising oxidant is air, while for synthetic fuels, alcohols (for instance methanol), hydrogen production or other syngas derived hydrocarbon products the gas providing the oxygen preferably essentially consists of oxygen.

The primary reformer would typically require a significant fired duty resulting in significant emissions of CO₂, when employed in a known process. By applying internal heat recovery in the reformer tubes, i.e. recuperative reforming, the fired duty per unit of syngas produced is reduced, leading to a reduced fired duty per produced volume of syngas; thus the same syngas production capacity can be maintained whilst reducing fired duty or one can increase syngas production capacity without increasing fired duty. The additional recovered duty can indeed be advantageously applied to reduce the carbon dioxide emissions from the primary reformer, by increasing the primary catalyst zone outlet temperature in the reformer and/or by increasing the throughput in the reformer reaction units (tubes) while maintaining the catalyst outlet temperature at par with conventional (pre-revamp situation) catalyst tubes. The recuperation part of the reforming reaction units typically lowers the outlet temperature of the primary reformate (7) by up to 250°C, thereby increasing the duty of the secondary reformer (9), when used. In the secondary reformer, the reformed syngas mixture from the primary reformer (7) is mixed with an oxidant (8), such as air (optionally enriched or depleted) or pure oxygen. The partial combustion of the syngas in the secondary reformer is highly exothermic and the outlet temperature of the secondary reformer should generally be 1100 °C or less, and preferably be in the range of 950-1050 °C, and is thereby a limiting factor in the design of secondary reformer: the higher the temperature at the inlet of the secondary reformer, the lower the reaction capacity of the secondary reformer to avoid exceeding the desired temperature due to the exothermal nature of the reaction.

A lower inlet temperature of primary reformate into the secondary reformer is achieved by applying the recuperative reformer reaction unit in the fired primary reformer, typically the inlet temperature into the secondary reformer in the range of 600-800°C; hereby the outlet temperature of the secondary reformer also decreases, when the oxidant/feed ratio is kept unchanged compared to a conventional process wherein no heat-recuperating primary reformer reaction is used. The lower outlet temperature of the primary reformer does allow the possibility to feed higher oxidant flowrate in the secondary reformer (9) (without exceeding the design limits of the secondary reformer).

However as the partial combustion of syngas (combustion reaction being a part of the autothermal reforming process) also combusts part of the hydrogen and the outlet temperature of the secondary reformer impacts the equilibrium of the synthesis gas. A lower outlet temperature will increase the methane slip which is an inert in the downstream process and a potential source of carbon-dioxide emissions.

In the present invention we apply in addition to the recuperative reforming step a parallel endothermal reaction (13), such as a parallel endothermal reforming step (13). The parallel reactor is generally located outside a radiant section, and thus non-fired. This allows for an increase in hydrogen production by means of the parallel (unfired) heat exchanger reactor (13). The parallel heat exchanger reactor can be applied for endothermic processes other than reforming, more specifically other high temperature endothermic process. Examples for such processes include: reverse water gas shift to produce carbon monoxide or syngas from hydrogen and carbon dioxide. Decomposition of ammonia for example for production of hydrogen, decomposition of methanol for example for production of hydrogen and/or syngas, fusel oil reforming to produce hydrogen. The heat required for these endothermic reactions is supplied by high temperature reformate from the primary or secondary reformer. The product from the endothermic reactions can either be combined with the primary or secondary reformate or send to a separate downstream process.

The reactants fed into the reactor depend on the intended reaction. The skilled person will be able to select different reactants for the parallel endothermal reaction, dependent on the intended reaction; e.g. in case of a reverse water gas shift reaction the reactions will comprise hydrogen and carbon monoxide; e.g. for ammonia cracking, the reactant will comprise ammonia.

In case of a parallel reformer reaction, hydrocarbon and further reformer reactant selected from steam, carbon dioxide and mixture thereof (elsewhere herein also referred to as 'second part') are used. Both the first part of the hydrocarbon feed (to the primary reformer) and the second part can be the same, e.g. a hydrocarbon stream from a single source can be split in the first and the second part. However, the composition of the part of the hydrocarbon feed into the parallel reactor can be the same as for the primary reformer, but does not have to be the same. It can, e.g. originate from different sources, such as a different hydrocarbon feedstock selected from those described elsewhere herein for the part of the hydrocarbon feed that is fed in to the primary reforming system.

In practice, it is usually convenient to use a mixture of hydrocarbon feed and further reformer reactant having essentially the same composition for feeding into the parallel reformer (13), as the mixture fed into the primary reformer. However, it is also possible to introduce a mixture having a different composition, e.g. a different hydrocarbon feed can be used, the ratio hydrocarbon feed to further reactant can be different or the further reactant can have a different composition. Steam is particularly suitable as a further reactant in a process according to the invention. Alternatively, instead of the addition of steam, part of the steam or the complete steam quantity can be replaced with carbon dioxide. Resulting in a reforming section feed essentially consisting of the hydrocarbon feedstock, carbon dioxide and optionally steam.

It is also possible to include another component, such as hydrogen, in one of the mixtures to be fed into the primary reformer and the parallel reformer or in both mixtures at the same or a different concentration.

With the implementation of a parallel reformer, the high temperature of the reformate exiting the secondary reformer (or if the secondary reformer is not employed: the primary reformer) provides the heat to the parallel reformer (generally at least for a substantial part by heat-conduction) and is thus used for producing more syngas and thereby unloading the fired duty of the primary reformer as well as decreasing the duty in the secondary reformer (when used) and subsequently lowering the CO₂ emissions of the plant.

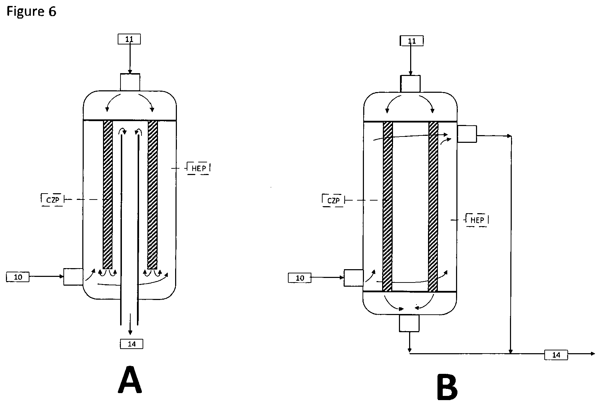

The parallel reformate or part thereof and the primary or secondary reformate or part thereof that has been used as heat exchange medium in the heat-exchanging medium passage way (HEP) are usually combined downstream of the parallel reformer catalyst zone (CZP) and downstream of the heat exchanging medium passage way (HEP), thereby forming a combined reformate gas (14). The combining can take place downstream of the parallel reformer catalyst zone, yet inside the parallel reformer unit or outside the parallel reformer unit. Figure 6A schematically shows a design for combining inside the parallel reformer. This makes the mechanical design simpler, but could require an increase in heat-exchange area due to lower temperature difference. Figure 6B schematically shows a design for combining inside the parallel reformer outside the reformer, which can add to the complexity of the design, but can reduce the required heat-exchange area.

The parallel heat exchanger reformer unit may be based on a heat exchanger known in the art, e.g. from WO2018/104526, of which the contents, in particular the claims and figures are incorporated herein by reference. A preferred heat exchanger reformer comprises a vessel having a plate assembly section placed therein comprising of several plates positioned at a distance from each other to provide at least alternating first and second channels between adjacent plates, which vessel comprises a first inlet at a first end of the plate assembly section for supplying a mixture of a hydrocarbon feed and further reformer reactant (steam, carbon dioxide) to the first channels and causing the mixture to flow in a direction toward a second end of the plate assembly section, which vessel also comprises a second inlet close to the second end of the plate assembly for supplying hot reformer effluent from the primary or secondary reformer as a heating gas flow to the second channels, wherein the second channels comprise a first and a second section which are connected to each other, wherein the first section is provided for conducting the hot reformer effluent in a direction towards the first end of the plate assembly counter current to the flow of the hydrocarbon feed and further reactant mixture in the first channels, and the second section is provided for conducting the hot reformer effluent to flow in cross direction of the first channels, which second channels are connected to a collector outlet for the reformer effluent to leave the heat exchanger reformer at the first end of the plate assembly.

The parallel heat exchanger reactor, such as a reformer, is installed outside (and in a parallel flow path for the mixture of hydrocarbon feed and further reactant) of the radiant section of the fired primary reformer system. Typically, when using a parallel reformer, 50 % or less of the total feed-further reactant mixture that is fed to the reformer system is fed through the reaction zone of the parallel reformer, whereas 50 % or more of the total feed-further reactant mixture is fed to the fired primary reformer. Heat for the reaction in the parallel (non-fired) heat exchanger reformer is supplied by primary/secondary reformer effluent, generally of at least 850 °C, in particular of 900 °C or higher, typically up to 1050 °C. Generally, the outlet temperature at the catalyst zone (bed) in the heat exchanger reformer unit is lower than the outlet temperature of the reformer of which the reformate is used as heat exchange medium for the reformer reaction in the parallel reformer, i.e. the secondary reformer, if used, or otherwise the primary reformer. This is due to the temperature difference (the driving force) necessary for the heat exchange process to take place The outlet temperature of the catalyst bed from the heat exchanger parallel reformer is typically in the range of 750 to about 950 °C, with the proviso that it is lower than the temperature of the reformate used as heat exchange medium at the outlet of the reformer it is taken from, usually at least about 20 °C lower. The pressure in the parallel reformer (22) outside the radiant section is generally about equal to the pressure in the fired reformer. Additional steam or other further reformer reactant (12) can be added at the feed of the parallel heat exchanger reactor to drive the reforming reaction towards hydrogen production. Thus, the ratio further reformer reactant (steam, carbon dioxide) to hydrocarbon feed, does not have to be the same in different reformer reaction units. Because the heat exchanger reformer is parallel to the fired reformer and reduces the required duty of the fired reformer (when comparing at equal syngas output), the reformer size can be reduced with the same throughput per tube. When using a parallel configuration, preferably about 10 to about 30 wt.% of the hydrocarbon feed, more preferably 15 - 25 wt.% is fed to the parallel (non-fired) heat exchanger reformer reaction unit. A higher split ratio will unload the reformer further, but reduce the driving force for the heat exchanger reformer, resulting in an increasingly larger parallel reformer exchanger.

The pressure in the reformer reaction units can be chosen within a known range, generally between 0.1 and 10 MPa, e.g. depending on the desired product pressure. Advantageously the pressure in a process according to the invention is at least 0.2 MPa, in particular at least 0.25 MPa. Usually, the pressure in the reformer reaction zone is 5 MPa or less, preferably 4 MPa or less, more preferably in the range of 0.2-4.0 MPa.

The available heat in the reformate (14) at the end of the reformer system (102), in particular the combined reformate (14), is usually further recovered with a process gas waste heat boiler (PGWH) (15) and the cooled reformate gas (16) is sent for further processing in the downstream units, depending on the application of the downstream syngas. Typically used units for downstream sections include one or more selected from the group consisting of water gas shift sections (103), cooling trains (104), CO₂ removal units (105), syngas purification units (such as units for recovering hydrogen or carbon monoxide) (106) and chemical product synthesis sections (107), such as a methanol production section or an ammonia production section. Such units and sections are generally known in the art and are selected based on the desired product or products (108) derived from the syngas produced in the reforming section. The CO₂ that has been captured can either be used in one of the other units or exported as a separate by-product (109) Further, these may be based on common general knowledge or the prior art cited herein above.

The invention can be reduced to practice by providing a newly built reformer system according to the invention. This allows carrying out a process according to the invention that operates at about the same or a lower pressure drop than a conventional scheme and therefore does not impact any downstream process. Also, the current invention can be applied for a capacity increase revamp scenario of an existing plant, without hydraulic limitation resulting from the increase flowrate through the equipment. Advantageously, a recuperative reforming technology that applies structured catalyst and thereby reduces the required pressure drop of the primary reformer is employed. This can compensate for the additional pressure drop of the increased throughput as well as the pressure drop of the shell of the parallel reformer. The total capacity of synthesis gas can therefore be increased significantly with similar or decreased pressure drop of the primary and secondary reformer, resulting in less hydraulic limitations for capacity increase revamps.

The present invention of combining both recuperative reforming in the firebox of the primary reformer with a heat exchanger reformer parallel to the primary and - when present the secondary - reformer allowing for cumulating the benefits of both separate technologies to significantly reduce the CO₂ emissions in the syngas production processes. Additionally, the combination of technologies also minimizes the drawbacks of the application of each technology applied separately.

One aspect of the present invention is the minimization of the total heat duty of the production system (methane to ammonia, methane to methanol etc...). The application of the present invention therefore also calls for the use of electrical power -more preferably power from renewable sources- for driving the machinery used for compression of the syngas upstream of the conversion reactor (methanol, ammonia etc.). Alternatively, the machinery can still be driven on very high pressure steam, by providing electric boilers or boilers heated by renewable energy (e.g. derived from biomass or hydrogen produced from renewable energy).

### Production of ammonia:

The process for producing ammonia typically comprises a syngas generation section and an ammonia synthesis section. The syngas generation section comprises the reformer system wherein hydrocarbon and steam are reacted and reformate streams are processed as described above. When producing syngas for ammonia production, generally use is made of a secondary reformer in the reforming section. Since nitrogen will be a reactant in the ammonia synthesis, the gas comprising oxidant that is supplied does not have to be pure oxygen. Advantageously air is used. The air flowrate to the secondary reformer is usually adjusted such that the H₂/N₂ ratio is about 3.0, which is the optimal (stoichiometric) ratio for the downstream ammonia synthesis loop. The secondary reformate or part thereof is usually combined with the parallel reformate or part thereof and then subjected to cooling in a waste heat boiler wherein steam is produced using heat from the reformate.

Further, the process for producing ammonia can be based on ammonia synthesis sections of plants and processes known per se, e.g. as described in the above cited prior art. The final reformate (typically the combined reformate) to be used for ammonia synthesis is typically fed into a shift reactor zone, wherein carbon monoxide reacts with water (steam) to form further hydrogen and carbon dioxide, resulting in a shift reactor process gas having an increased hydrogen and carbon dioxide content and a reduced CO content, compared to the reformate. The shift reactor process gas to be used for ammonia synthesis is generally subjected to one or more processing steps wherein the carbon oxides are essentially removed from said process gas, by which one or more processing steps a hydrogen-enriched process gas is obtained. The carbon oxides removal is adequately accomplished, e.g., by first capture of carbon dioxide (e.g. with an amine unit) and subsequently a methanation of residual CO. Thus a hydrogen-enriched process gas is obtained (compared to the shift reactor process gas), which is also referred to as raw synthesis gas. The methane present in the raw synthesis gas is an inert in the downstream ammonia synthesis loop and is therefore preferably minimized. Usually the raw synthesis gas fed into the ammonia synthesis loop comprises at least 65 vol % hydrogen. After compression of the raw synthesis gas to - typically - 150-200 barg, it is fed to the ammonia synthesis section where ammonia is produced. Remaining synthesis gas is recycled to the feed of the ammonia reactor, thus forming a so called ammonia loop. The inert methane in the loop is removed through a small purge stream and typically used as a fuel in the primary reformer.

### Production of methanol:

In accordance with the invention, methanol can be produced from carbon monoxide (and optionally carbon dioxide) and hydrogen by a catalytic reaction in a methanol reactor, wherein at least part of the carbon oxide and at least part of the hydrogen are provided by the reformate that has been used as heat exchange medium in the parallel reformer (13), from the parallel reformate or from the combination of said reformates. In a preferred embodiment, the primary reformate or part thereof and oxygen (preferably >95 vol %, more preferably >97.5 mol %; up to 100 %, in particular up to 99.9 %) are fed into the secondary reformer (9) wherein the primary reformate is reacted with the oxygen, whereby the secondary reformate is formed. The secondary reformate (10) or part thereof is fed into the heat-exchanging medium passage way (HEP) where it transfers heat to the parallel reformer catalyst zone (CZP). Parallel reformate and secondary reformate are advantageously thereafter combined and wherein at least part of said hydrogen and at least part of said carbon monoxide are used for said production of methanol. For methanol synthesis, reformate comprising carbon oxide (CO and optionally CO₂) mixture and hydrogen is usually fed to the methanol synthesis loop without treatment in a shift section and without treatment in a CO/CO₂ removal section. Although, if desired, in particular a treatment in a shift reactor is applied. Optionally a CO₂ removal unit can be present, though typically this is not required for methanol synthesis. Typically the process gas to be used as a feed into the methanol synthesis section is cooled down and the condensed water is separated from the process gas before feeding it into the methanol synthesis section.

Other products synthesized from syngas, from a component of syngas or from a chemical produced from syngas or a component thereof can be produced based on synthesis equipment and process conditions known *per se.* E.g. dimethylether (DME) is a direct and cleaner alternative for diesel and an important chemical intermediate for further chemical production. The production can be based, for example, on US20070078285A1, which teaches a method to produce DME from syngas and/or methanol.

In summary, the present invention in particular provides one or more of the following advantages:
A first main advantage of the invention is that it allows significant increase of the syngas from the reforming section for the same fired heat input(> 20% Up to 70%) in syngas plants (most notably up to 50% ammonia plants), while maintaining the optimal in the synthesis gas ratio (H2/N2 ratio in case of ammonia production). A reduction of ton hydrocarbon (feed+ fuel) per ton syngas produced of up to 15% can be achieved, while also decreasing the amount of fuel fired at the same time. This therefore results in significant reduction of CO₂ emissions from the syngas production section. The invention thus results for lower CO₂ emissions per syngas produced (up to 40%).

As the throughput of the reforming section is significantly increased, a small part of the excess syngas produced in the form of hydrogen can be extracted from the downstream syngas treatment section and used as make up fuel in order to reduce the CO2 emissions from the reforming section even further. Combined with recycling hydrocarbon purge from the synthesis loop more than 95% of CO2 emissions could be avoided without an increase in reforming section and maintaining the benefits of reduced firing and hydrocarbon consumption as stated above.

Secondly, the invention of a combination of recuperative reforming in the primary reformer and Parallel heat exchanger reformer parallel to the primary and - if applicable - secondary reformer combines the benefits of both separate technologies and eliminates the downsides of either technology. The recuperative reforming in the primary reforming before the gas flows to the secondary reformer where it reacts with an oxidant, can decrease the methane slip which is typically an inert downstream, this can also compensate for a higher methane slip from a parallel reformer exchanger.

Another important benefit of the present invention is that the combination of recuperative reforming in the primary reformer, a secondary reformer and parallel heat exchanger reformer unit is that that is a large range of syngas ratios that can be achieved within the same unit. This enables production of syngas tailored to the downstream users. For example in case of methanol production, the optimum stoichiometric is approximately 2.0, while for other process this can be either higher or lower. By adjusting the split to the parallel heat exchanger reformer as well as the oxidant to the secondary reformer and steam to the primary reformer a large range of H2 to CO ratio is possible and provides additional flexibility over existing processes. It also allows for the possibility to produce additional hydrogen in the syngas unit which as a slip stream can be extracted from the syngas (for example by a membrane) and supplied to other users. For example, to supply the supplemental hydrogen as a fuel in fired heaters thereby eliminating the carbon dioxide emissions from burning fossil fuels. The flexibility in the produced syngas ratios can be tailored by adjusting the split between the primary reformer and the parallel heat exchanger reformer. A larger split to the parallel reformer increases the H2 to CO ratio, while a smaller split decreases the H2 to CO ratio. This flexibility in H2 to CO ratios in produced syn gas gives a lot of flexibility in deciding the design parameters for downstream synthesis section such as methanol loop parameter selections and desired recycle ratios. The present invention can thus supply syngas in the optimized ratio for multiple downstream processes opening an attractive path to hybrid plants (producing methanol, ammonia, hydrogen product and/or or other syngas derived products).For example if more methanol production is desired the syngas ratio can be adjusted to comply with that requirement, while if less methanol is required compared to the other product(s) the syngas ratio is optimized accordingly.

The same can also apply for the production of syngas from multiple feedstocks or feedstocks that significantly vary in composition. The syngas H₂ to CO ratio depends on the feedstock and with the flexibility in the present invention the syngas ratio can easily be optimized to achieve the optimum condition for the downstream synthesis loop. This can be especially attractive in case of bio-based feedstocks or off gasses which usually tend to vary significantly in quantity as well as composition.

Fourthly, for the same syngas production, a smaller primary reformer (Typically up to 50% smaller) would be required thereby reducing the required footprint of the plant. Surprisingly, the required reformer size reduces more when recuperative reforming is combined with parallel heat exchanger reformer than the additional of the savings by both technologies separately.

Alternatively the same advantage of the invention is that this enables significant capacity increase or decarbonization of existing syngas producing facilities with only minor changes in the reforming section. The recuperative reforming technology can be applied in the existing radiant tubes of the primary reformer, while the parallel reformer is a compact heat exchange reformer with a small footprint. The downstream process gas boiler duty will be reduced so minimum modifications are required.

Additionally, the pressure drop of the present invention is lower or similar to the conventional production route for syngas. When combining the recuperative reforming with structured catalysts, results in a lower pressure drop than a conventional reformer, the secondary reformer pressure drop will be similar while the parallel heat exchanger reformer is operated in parallel and only the shell side requires a small amount of pressure drop. This combination results in a pressure drop equal or lower than a conventional production route with primary and secondary reformer. The main advantage of the lower pressure drop is that this enables a reduction of the compression requirement of the syngas compressor (if applicable) resulting in additional savings of power and thus carbon footprint (either for generating the steam driving the turbines of the compressor or for generating the power for the motors driving the compressor). The invention thus reduces the CO₂ reduction further than only the direct CO₂ emission reduction of the syngas production side. In case of a capacity revamp scenario as discussed above this lower pressure drop mitigates a major part of the hydraulic limitations for capacity expansion and therefore allowing a significantly larger capacity increase than alternative solution for increasing the pressure drop of a syngas production unit.

The present combination can also efficiently combined with electrification of syngas production. The heat required for the primary reformer (including the recuperative reforming) can be applied by either firing of purge gasses from the downstream process and/or a make-up fuel (such as natural gas or hydrogen) or by electrified reformer. The electrification basically replaces fired heater to supply the heat for reforming reaction and can result in zero direct CO₂ emissions.

Next the invention is illustrated by some examples.

### Example 1 ammonia production

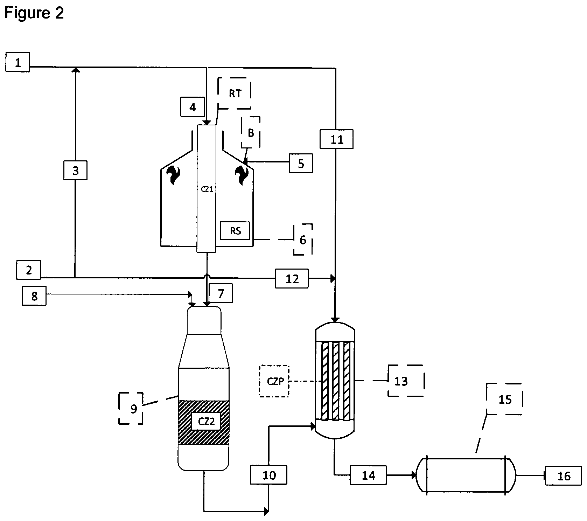

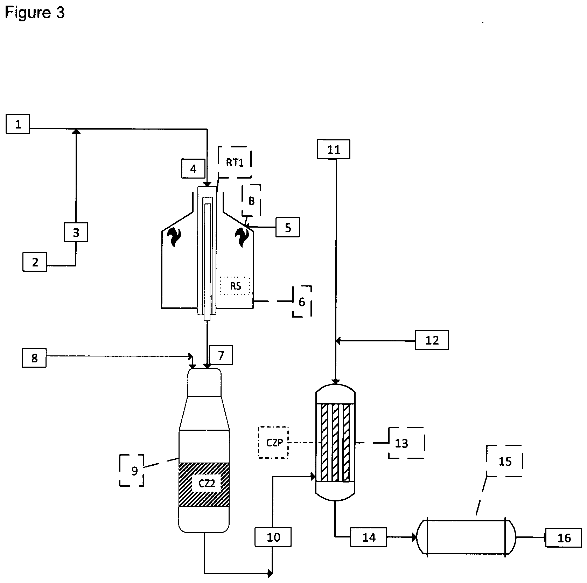

As an example, supporting the invention, four ammonia production cases were compared with each other. Case 1 (Figure 1) is typical ammonia plant producing ∼50 t/h of ammonia with the traditional ammonia scheme (with a primary fired reformer and a secondary reformer, but without parallel reformer.
Case 2 (Figure 2) is as in Case 1 but with an additional parallel heat exchanger reformer parallel to the primary and secondary reformer. The heat exchanger reformer uses the hot effluent of the secondary reformer.
Case 3 is as Case 1, with the proviso that the convention reformer catalyst tubes in the fired primary reformer have been replace with heat-recuperative reforming tubes.
Case 4 (Figure 3) is according to the invention with a parallel heat exchanger reformer parallel to the primary and secondary reformer as well as recuperative reforming in the primary reformer.
In all cases the feed as well as make-up fuel in the primary reformer are the same hydrocarbons feedstock and main operating conditions are kept equal (steam to carbon ratio, inlet temperature of primary reformer, catalyst outlet temperature, temperature of air to secondary reformer) and the outlet temperature of the secondary reformer is limited to 1000°C. The results are indicative and with optimization of the different parameters further improvements can be achieved. The focus of these results is on the production of the syngas (the combined reformate); the impact downstream is described qualitatively only.

| | Case 1 | Case 2 | Case 3 | Case 4 |
|---|---|---|---|---|
| HC consumption/ NH₃ produced [t/t] | base | -2% | -2% | -9% |
| CO₂ captured/ NH₃ exported [t/t] | base | +4% | +2% | +1% |
| CO₂ emission/ NH₃ produced [t/t] | base | -15% | -15% | -35% |
| Primary reformer size | Base | -12% | -10% | -32% |
| Steam produced | Base | -18% | -18% | -40% |
| H₂/N₂ ratio of the raw synthesis gas | ∼3.0 | ∼3.2 | ∼3.0 | ∼3.0 |
| Methane slip from reforming section [vol% (dry)] | 0.27 | 0.21 | 1.08 | 0.25 |

As can be derived from the table above, the addition of a parallel heat exchanger reformer (case 2) can reduce the required firing and thus the CO₂ emissions significantly. Also the methane slip can be reduced, depending on the outlet temperature and steam added to the process. However as the outlet temperature of the secondary reformer is limited, the H₂/N₂ ratio increases above the optimum ratio for the downstream ammonia synthesis loop. To maintain the optimum ratio additional nitrogen (from for example an air separation unit) should be added downstream the reforming section, adds to the size of the plant and complexity of the process. As a result of this limitation the split if often limited to 10-15% of the feed to the parallel heat exchanger reformer.

Case 2 with recuperative reforming also gains some benefits and can keep the H₂/N₂ the same or lower, depending on the selected operating conditions. The main observation is that the outlet temperature of the recuperative reformer is lower, which results in a lower outlet temperature of the secondary reformer. Subsequently the methane slip of the secondary reformer is increased. Alternatively additional oxidant can be supplied to the secondary reformer, this would result in a higher outlet temperature and thus a lower methane slip, but will also combust more hydrogen. Subsequently the H2/N2 ratio will decrease to -2.3 mol/mol. This is a downside of the recuperative reforming for the syngas production in case of ammonia production. The benefits are that it significantly reduces CO₂ emissions and thus hydrocarbon consumption as well as the primary reformer size.

Surprisingly, the current invention (case 4) combines the positive effects of both recuperative reforming and parallel heat exchanger reformer while eliminating the downsides. A significant reduction of hydrocarbon consumption can be achieved, larger than the sum of the reductions achieved in Cases 2 and 3 (9% vs. 5%) can be achieved, while reducing the CO₂ emission by 35% (vs. 30 % for the sum of Cases 2 and 3). This all can be done with a more than 30% smaller primary reformer. This can thus also be an attractive solution to increase the capacity of existing assets as well as for increasing the energy efficiency of existing assets.

It is further noteworthy that the methane slip in Case 4 is kept at a level below the methane slip of Case 1 (whereas employing heat recuperative reforming in the primary reformer a such leads to a fourfold increase in methane slip, see Case 3).

Though results for other syngas application do not have a limitation on the H₂/N₂ ratio, similar conclusions are also valid for the present invention.

### Example 2 methanol production

As an example for methanol production where in the art typically for small plants (<2000MTPD methanol) SMR is applied, mid size (<5000MTPD methanol) an SMR+ATR and large plants (>5000MTPD methanol) ATR only is applied due to economic and mechanical considerations the following. In case of methanol production, oxygen from an air separation unit (ASU) is typically applied as oxidant on large capacity plants. Similar to the example with ammonia production, in Case 1(Figure 1) the reformer section consists of a primary fired reformer and a secondary reformer, but without parallel reformer.
Case 2 (Figure 2) is as in Case 1 but with an additional parallel heat exchanger reformer parallel to the primary and secondary reformer. The heat exchanger reformer uses the hot effluent of the secondary reformer.
Case 3 is as Case 1, with the proviso that the convention reformer catalyst tubes in the fired primary reformer have been replace with heat-recuperative reforming tubes.
Case 4 (Figure 3) is according to the invention with a parallel heat exchanger reformer parallel to the primary and secondary reformer as well as recuperative reforming in the primary reformer.
Case 5 is based on a autothermal reformer (ATR) only (e.g. only a secondary reformer).

| | Case 1 | Case 2 | Case 3 | Case 4 | Case 5 |
|---|---|---|---|---|---|
| Oxygen consumption | base | -15% | +11% | -17% | +48% |
| Direct CO₂ emission | base | -16% | -22% | -43% | -90% |
| Stoichiometric number R | ∼2.2 | ∼2.3 | ∼2.1 | ∼2.2 | ∼1.8 |
| Methane slip from reforming section [vol% (dry)] | ∼0.2 | ∼0.3 | ∼0.2 | ∼0.2 | ∼0.1 |
| Primary reformer size | Base | -14% | -20% | -40% | Not applicable |

The results in the table are based on an equal syngas production. Typically an SMR+ATR scheme (case 1) is preferred over the ATR only scheme (case 50 as the SMR+ATR schemes can result in an optimum stoichiometric ratio (R) of ∼2.0. The use of an ATR only typically results in a deficit of hydrogen (R=∼1.8) and thus has a sub-stoichiometric ratio resulting in the downstream methanol synthesis loop operating outside the optimal range, while with the current invention the optimum stoichiometric number can be maintained. In a conventional SMR+ATR methanol plant the capacity is typically limited by the primary reformer size at ∼5000MTPD methanol and for larger capacities therefore ATR only schemes are selected. From the table above is shown that with the present invention, for the same reformer size >40% more capacity can be achieved and thus capacities up to ∼7500MTPD methanol can be achieved.

An additional benefit of the present invention is that the oxidant consumption for the same product capacity is -80% less oxidant consumption compared to an ATR only case and thus a smaller air separation unit (and resultingly lower power consumption).

The present invention can also avoid downsides of applying either recuperative reforming or parallel heat exchanger reforming separately. In case of the parallel heat exchanger reformer, the methane slip as well as the R-value typically increase, while with the recuperative reforming both decrease. The combination of both technologies in the present invention allow for balancing both effects and optimizing both of the values.

Also the current invention results in a reduction of oxygen required to reach conversion similar to case 2, while the recuperative reforming technology typically requires additional oxygen, ultimately leading to ∼17% less oxygen required compared to the base case.

### LEGEND TO FIGURES

1 = hydrocarbon feed
2 = further reformer reactant (steam, carbon dioxide or mixture of steam and carbon dioxide)
3 =further reformer reactant (steam, carbon dioxide or mixture of steam and carbon dioxide)
4 = hydrocarbon and further reformer reactant mixture (into primary reformer reaction unit)
5 = fuel (inlet to burners)
6 = fired primary reformer
7 = primary reformate (into secondary reformer reaction unit)
8 = oxidant (into secondary reformer reaction zone)
9 = secondary reformer (for example ATR, POX)
10= secondary reformate (into heat-exchange medium passage way of parallel reformer)
11= hydrocarbon and further reformer reactantmixture (into parallel reformer reaction unit)
12= additional steam, carbon dioxide or mixture thereof (for reaction with hydrocarbon in parallel reformer)
13= parallel process-gas heated reformer
14= combined reformate
15= process gas waste heat boiler (PGWH)
16= combined reformate to downstream processing, e.g. shift section, cooling train, CO₂ removal, ammonia reactor, methanol reactor, hydrogen recovery unit.

30= feed inlet for hydrocarbon and further reformer reactant mixture (4) into primary reformer reaction unit
31= outlet for primary reformate from primary reformer catalyst zone
32= primary reformate passage way ((inner channel; in Figure 4b: upstream part of the reformate passage way) arranged to transfer heat by conduction from primary reformate to the contents of the primary reformer catalyst zone (CZ1) (via a partition P)
33= primary reformate outlet out of the primary reformer reaction unit
34= downstream part of primary reformate passage way (inner channel)

RS= radiant section of primary reformer 6.
RT= primary reformer reaction unit
RT1= primary heat recuperating reformer reaction unit (located in the radiant section RS)
CZ= reformer catalyst zone in primary reformer reaction unit
CZ1= reformer catalyst zone in primary heat recuperating reformer reaction unit
B = burners in radiant section of the primary reformer
P = partition (wall) between primary reformer catalyst zone (outer channel) of primary reformer and primary reformate passage way (part) 32 arranged to transfer heat to primary catalyst zone
CZ2= Secondary reformer catalyst zone
CZP= Parallel process-gas heated reformer catalyst zone

101=Hydrocarbon feed purification section (e.g desulphurization, olefin saturation)
102= Reforming section (see figure 1 to 3 for detailed flow scheme of reforming section)
103= Shift reactor zone
104= Cooling train
105= CO₂ removal unit
106= syngas purification unit (e.g. hydrogen recovery unit or carbon monoxide recovery unit)
107= chemical synthesis loop (e.g. methanol synthesis, ammonia synthesis, GTL loop)
108= Product (e.g. hydrogen, carbon monoxide, ammonia, methanol)
109= CO₂ stream for export (high purity)

## Claims

1. A process for producing a chemical product from a hydrocarbon feed (1) and a further reforming reactant (2,3) selected from the group consisting of steam, carbon dioxide and mixtures thereof, the process comprising an endothermal reaction in a reformer reaction system, which reformer reaction system comprises a primary fired reformer (6), wherein the primary reformer (6) is a fired heat-recuperating reformer, comprising a radiant section (RS) provided with burners (B), wherein fuel (5) is combusted to provide heat to at least a first part (4) of the hydrocarbon feed and further reformer reactant, and a primary heat-recuperating reformer reaction unit (RT1), located in the radiant section (RS),
which primary reformer reaction unit (RT1) comprises a primary reformer catalyst zone (CZ1), containing a reformer catalyst, into which catalyst zone the hydrocarbon feed or said first part thereof and the further reformer reactant or said first part thereof are fed and in which catalyst zone the fed hydrocarbon and further reformer reactant react in the presence of the catalyst, whereby a primary reformate is formed, which primary reformer reaction unit (RT1) further comprises a primary reformate passage way (32, 34), downstream of the primary reformer catalyst zone (CZ1), through which passage way (32, 34) the primary reformate passes and which passage way (32, 34) is arranged to transfer heat inside the primary reformer reaction unit (RT1) from the primary reformate whilst present in the primary reformate passage way (32, 34) to the primary reformer catalyst zone (CZ1) before the primary reformate leaves the primary reformer reaction unit via a primary reformate outlet (33);
optionally subjecting the primary reformate to a secondary reforming reaction, thereby forming a secondary reformate;
using primary reformate or second reformate as a heat exchange medium to supply reaction heat to an endothermal reaction, which endothermal reaction is carried out in a parallel heat-exchanger reactor (13), which may be a heat-exchanger reformer reactor wherein a second part of the hydrocarbon feed and a second part of the reformer reactant are fed (11) or which parallel heat-exchanger reactor (13) is another heat-exchanger reactor configured for a different endothermal reaction than an endothermal reformer reaction and wherein one or more reactants are fed for said different endothermal reaction, wherein the parallel heat-exchanger reactor (13) comprises an endothermal reaction zone (CZP) wherein an endothermal reaction is carried out and a heat-exchanging medium passage way (HEP) through which the primary or secondary reformate passes and which passage way is arranged to transfer heat from the reformate present in the heat-exchanging medium passage way (HEP), to the parallel endothermal reaction zone (CZP) inside the parallel heat-exchanger reactor (13).

2. Process according to claim 1, wherein the parallel endothermal reaction is selected from the group consisting of reforming reactions; reverse water gas shift reactions to produce carbon monoxide or syngas from hydrogen and carbon dioxide; ammonia decomposition, for example for production of hydrogen; methanol decomposition; for example for production of hydrogen and/or syngas; and fusel oil reforming to produce hydrogen.

3. Process according to claim 1 or 2, wherein a first part of the hydrocarbon feed and a first part of the further reforming reactant are fed (4) to the primary heat-recuperating reformer (6) a second part of the hydrocarbon feed and a second part of the further reforming reactant, are fed (11) to the parallel reactor (13), wherein the parallel reformer is a heat-exchanger reformer (13), comprising a parallel reformer catalyst zone (CZP) into which the second part of the hydrocarbon feed and the second part of the further reforming reactant (11), plus optionally additional further reformer reactant (12), are fed, and in which parallel reformer catalyst zone (CZP) the fed hydrocarbon and further reformer reactant - react in the presence of a reformer catalyst present in the catalyst zone (CZP) whereby a parallel reformate is formed,
the parallel reformer (13) further comprising a heat-exchanging medium passage way (HEP), arranged to transfer heat from a heat exchange medium present in the heat-exchanging medium passage way (HEP), to the parallel reformer catalyst zone (CZP) inside the parallel reformer (13), wherein the heat exchange medium is primary reformate (7) from the primary reformer or a secondary reformate (10), obtained after subjecting primary reformate to further processing, in particular a secondary reforming reaction.

4. Process according to claim 3, wherein the part of the hydrocarbon fed to the primary reformer has about same composition as the hydrocarbon fed to the parallel reformer.

5. The process according to any of the preceding claims, wherein the primary reformate or part thereof and a gas comprising an oxidant (5), preferably a gas comprising oxygen as the oxidant, are fed into a secondary reformer (9) and are subjected to a secondary reforming reaction in the secondary reformer (9) wherein the primary reformate reacts with the oxidant, whereby a secondary reformate is formed and feeding the secondary reformate (10) or part thereof into the heat-exchanging medium passage way (HEP) where it transfers heat to the parallel endothermal reaction zone, preferably a parallel reformer catalyst zone (CZP).

6. The process according to claim any of the claims 2-5, wherein in the endothermal reaction zone a parallel gas stream is formed from the one or more endothermal reactants, preferably a parallel reformate and the parallel gas stream or part thereof and primary reformate or part thereof respectively secondary reformate or part thereof that has been used as heat exchange medium in the heat-exchanging medium passage way (HEP) are combined downstream of the parallel reformer catalyst zone (CZP) and downstream of the heat exchanging medium passage way (HEP), thereby forming a combined reformate gas (14).

7. The process according to any of the preceding claims, wherein at least ammonia is produced from nitrogen and hydrogen by a catalytic reaction in an ammonia reactor, wherein at least a part of the hydrogen is provided by the secondary reformate that has been used as heat exchange medium in the parallel reformer (13) or by the combination of secondary reformate and parallel gas stream, which parallel stream preferably is parallel reformate (14).

8. The process according to claim 7, wherein at least a part of said secondary reformate or at least part of said combination of secondary reformate and parallel gas stream, preferably parallel reformate, is fed to a shift reactor zone, thereby forming a shift reactor process gas having a reduced CO content, an increased CO₂ content and an increased hydrogen content compared to the reformate that is fed to the shift reactor;
subjecting at least part of the shift reactor process gas to one or more processing steps wherein the carbon oxides are essentially removed from said process gas, by which one or more processing steps a hydrogen-enriched gas is obtained and the hydrogen-enriched gas or a part thereof are fed into an ammonia synthesis reactor, wherein the hydrogen is reacted with nitrogen, which may be nitrogen present in the hydrogen-enriched gas or additionally supplied nitrogen, in the presence of an ammonia catalyst thereby forming ammonia.

9. The process according to claim 7 or 8, wherein the primary reformate or a part thereof is subjected to a secondary reforming reaction in a secondary reformer (9), in which secondary reformer primary reformate is contacted with air, providing oxygen and nitrogen,
the primary reformate in the secondary reformer reacts with the oxygen, whereby a secondary reformate is formed,
feeding the secondary reformate (10) or a part thereof into the heat-exchanging medium passage way (HEP) where it transfers heat to the parallel reformer catalyst zone (CZP),
combining at least a part of the secondary reformate that has been used as the heat-exchanging medium and the parallel reformate downstream of the parallel reformer catalyst zone (CZP) and downstream of the heat exchanging medium passage way (HEP), thereby forming combined reformate gas, comprising hydrogen and nitrogen originating from said air, and wherein said hydrogen or a part thereof and said nitrogen a part thereof are fed to the ammonia reactor as reactants.

10. The process according to any of the preceding claims wherein at least methanol is produced from carbon monoxide and hydrogen by a catalytic reaction in a methanol reactor, wherein at least part of the carbon monoxide and at least part of the hydrogen are provided by the reformate that has been used as heat exchange medium in the parallel reformer (13), from a parallel reformate or from the combination of said reformates.

11. The process according to claim 10, wherein the primary reformate or part thereof and a gas comprising oxygen, preferably a gas at least substantially consisting of oxygen, are fed into a secondary reformer (9) wherein the primary reformate is reacted with the oxygen, whereby a secondary reformate is formed, feeding the secondary reformate (10) or part thereof into the heat-exchanging medium passage way (HEP) where it transfers heat to the parallel endothermal reaction zone (CZP),
combining the secondary reformate or part thereof and the parallel gas stream, preferably parallel reformate, or part thereof downstream of the parallel endothermal reaction zone (CZP) and downstream of the heat exchanging medium passage way (HEP), thereby forming combined reformate gas, comprising hydrogen and carbon monoxide, and wherein at least part of said hydrogen and at least part of said carbon monoxide are used for said production of methanol.

12. The process according to any of the preceding claims, wherein at least hydrogen is obtained, in which process at least part of the parallel gas stream, preferably parallel reformate, at least part of the primary respectively secondary reformate that has been used as heat exchange medium in the parallel reactor (13) or at least part of the combination of said reformates is used to recover hydrogen from, or wherein at least part of the parallel gas stream, preferably parallel reformate, at least part of the primary respectively secondary reformate that has been used as heat exchange medium in the parallel reformer (13) or at least part of the combination of said reformates is fed to a shift reactor zone, wherein a shift reactor a process gas having an increased hydrogen content compared to the fed reformate is formed, and hydrogen is recovered from the shift reactor process gas.

13. The process according to any of the preceding claims, wherein at least syngas is obtained as a product.

14. The process according to any of the preceding claims, wherein primary reformate, secondary reformate, parallel gas stream, a combination of parallel reformate and primary reformate or secondary reformate, syngas obtained from any of said reformates, hydrogen obtained from any of said reformates, carbon monoxide obtained from any of said reformates, produced ammonia or produced methanol is used for the production of another product of interest, e.g. a synthetic fuel, dimethylether, or an alcohol having at least two carbon atoms, which alcohol having at least two carbon atoms preferably is a C2-C10 alcohol.

15. The process according to any of the preceding claims, wherein the primary reformer catalyst zone (CZ1) is present in an outer reactor channel and the primary reformate passage way (32, 34) is or forms part of an inner channel, configured to exchange heat with the outer reactor channel, said heat recovery extending coaxially inside the outer reactor channel, the outer reactor channel containing a catalyst bed catalysing the reaction between the hydrocarbon and the water under formation of the primary reformate, wherein the outer reactor channel has a feed inlet (30) via which the hydrocarbon and the water are fed through the catalyst bed and an outlet (31) for primary reformate, which inlet and outlet are located at opposite ends of the catalyst bed, wherein the primary reformate is fed from outlet (31) into the inner channel (32, 34), heat is transferred from the primary reformate flowing through the inner channel to the contents of the outer reactor channel, and primary reformate leaves the primary heat-recuperating reformer unit (RT1) via a gas outlet (33).

16. The process according to any of the preceding claims, wherein the further reformer reactant fed to the primary reformer and/or the further reformer reactant fed to the parallel reformer is steam or at least substantially consists of steam.

17. The process according to any of the preceding claims, wherein the further reformer reactant fed to the primary reformer and/or the further reformer reactant fed to the parallel reformer is carbon dioxide or at least substantially consists of carbon dioxide.

18. Chemical plant, comprising a reformer reaction system for producing a product from a hydrocarbon feed (1) and a further reformer reactant, (2), such as steam or carbon dioxide, the reformer reaction system, comprising a primary reformer (6), the plant further comprising a parallel heat-exchanger reactor configured to carry out an endothermal reaction (13), preferably a heat-exchanger reformer (13),
wherein the primary reformer (6) is a fired heat-recuperating reformer, comprising a radiant section (RS), wherein burners (B) and a primary reformer reaction unit (RT1) are present, which primary reformer reaction unit (RT1) comprises a primary reformer catalyst zone (CZ1), containing a reformer catalyst and an inlet for feeding hydrocarbon feed and further reformer reactant into the primary reformer catalyst zone (CZ1), which primary reformer reaction unit (RT1) further comprises a primary reformate passage way (32, 34), downstream of the primary reformer catalyst zone (CZ1), configured to receive primary reformate formed in the primary catalyst zone and allow passage of said primary reformate through the passage way (32, 34) and which passage way (32, 34) is arranged to internally transfer heat from the primary reformate in said passage way (32, 34) to the primary reformer catalyst zone (CZ1) and a primary reformate outlet (33) downstream of the passage way (32, 34) arranged to internally transfer heat to the primary reformer catalyst zone (CZ1); and wherein
the parallel heat-exchanger reactor (13), comprising a parallel reaction zone, preferably a catalyst zone (CZP), comprising an inlet for reactants, e.g., hydrocarbon feed and steam, carbon dioxide or a mixture thereof (11), plus optionally an additional reactant inlet (12),
the parallel heat exchanger reactor (13) further comprising a heat-exchanging medium passage way (HEP), arranged to transfer heat from a heat exchange medium present in the heat-exchanging medium passage way (HEP), to the parallel reaction zone (CZP) inside the parallel heat exchanger reactor (13) wherein the heat exchange medium passage way has an inlet for heat exchange medium in fluid communication with the outlet (33) of the primary reformer reaction unit via a passage way (7, 9, 10), which optionally contains one or more processing units, e.g. a further reformer, configured to treat primary reformate, e.g. process primary reformer into a secondary reformate (10), obtained after subjecting primary reformate to further processing, in particular a secondary reforming reaction.

19. Chemical plant according to claim 18, wherein the reformer reaction system further comprises a secondary reformer (9), the secondary reformer (9) comprising a secondary reformer reaction zone configured to produce a secondary reformate, the secondary reformer reaction zone comprising an inlet which is in fluid communication with the primary reformate outlet (33) via a passage way (7), the secondary reformer reaction zone further comprising an inlet for oxidant gas (8) and an outlet for secondary reformate which outlet is connected via a passage way (10) to the inlet into the heat-exchanging medium passage way (HEP) of the parallel reformer (13), the plant further comprising a provision for combining the secondary reformate and the parallel reformate downstream of the heat-exchanging medium passage way (HEP) and the parallel reformer catalyst zone (CZP) of the parallel reformer (13).

20. Chemical plant according to claim 18 or 19, wherein the primary reformer catalyst zone (CZ1) is present in an outer reactor channel and the primary reformate passage way (32, 34) is or forms part of an inner channel, configured to exchange heat with the outer reactor channel, said heat recovery extending coaxially inside the outer reactor channel, the outer reactor channel containing a catalyst bed configured for catalysing the reaction between hydrocarbon and water under formation of the primary reformate, wherein the outer reactor channel has a feed inlet (30) configured for feeding hydrocarbon and water through the catalyst bed and an outlet (31) for primary reformate, which inlet and outlet are located at opposite ends of the catalyst bed, wherein the outer channel has outlet (31) in fluid communication with the inner channel (32, 34), which inner channel is configured to transfer heat from the primary reformate flowing through the inner channel to the contents of the outer reactor channel.

21. Chemical plant according to any of the claims 18-20, wherein the plant is a hybrid chemical plant, configured to obtain at least two different chemical products from a reformate stream of the reformer reaction system, wherein the at least two different chemical products are selected from the group consisting of ammonia, methanol, hydrogen, carbon dioxide, carbon monoxide, synthetic fuel, dimethylether(DME) and higher alcohols.
